Europäisches Patentamt

European Patent Office

Office européen des brevets

(19)

(11) Veröffentlichungsnummer : **0 550 455 B1**

# (12) EUROPÄISCHE PATENTSCHRIFT

(45) Veröffentlichungstag der Patentschrift :
**10.08.94 Patentblatt 94/32**

(21) Anmeldenummer : **91914767.8**

(22) Anmeldetag : **21.08.91**

(86) Internationale Anmeldenummer :
**PCT/EP91/01590**

(87) Internationale Veröffentlichungsnummer :
**WO 92/05117 02.04.92 Gazette 92/08**

(51) Int. Cl.$^5$ : **C02F 3/34,** C02F 1/46,
C02F 1/70, C02F 1/72

(54) **VERFAHREN ZUR WASSERREINIGUNG.**

(30) Priorität : **26.09.90 DE 4030488**

(43) Veröffentlichungstag der Anmeldung :
**14.07.93 Patentblatt 93/28**

(45) Bekanntmachung des Hinweises auf die
Patenterteilung :
**10.08.94 Patentblatt 94/32**

(84) Benannte Vertragsstaaten :
**DE FR GB**

(56) Entgegenhaltungen :
**EP-A- 068 582
FR-A- 2 310 974
US-A- 756 832
WORLD PATENTS INDEX LATEST WEEK 4149.
Derwent Publications Ltd. ,Lodon, GB. AN
83-09295K
WORLD PATENTS INDEX LATEST WEEK
449,Derwent Publications Ltd. London, GB, AN
90-144903**

(73) Patentinhaber : **MOBITEC MOLECULAR
BIOLOGISCHE TECHNOLOGIE GMBH
Wagenstieg 5
D-37077 Göttingen (DE)**

(72) Erfinder : **MELLOR, Robert, B.
Frederiksborgvej 40,st.tv.
DK-4000 Roskilde (DK)**
Erfinder : **RONNENBERG, Jörg
Am Wall 18
D-3401 Seeburg (DE)**
Erfinder : **DIEKMANN, Stefan
Ulmenweg 2
D-3406 Bovenden (DE)**

(74) Vertreter : **Huber, Bernhard, Dipl.-Chem. et al
Patentanwälte
H. Weickmann, Dr. K. Fincke
F.A. Weickmann, B. Huber
Dr. H. Liska, Dr. J. Prechtel, Dr. B. Böhm
Postfach 86 08
20
D-81635 München (DE)**

EP 0 550 455 B1

EP 0 550 455 B1

## Beschreibung

Die vorliegende Erfindung betrifft ein Verfahren zur Umsetzung reduzierbarer oder oxidierbarer Substanzen in wäßriger Lösung.

Aufgrund der zunehmenden Umweltbelastung wird die Bereitstellung von reinem Wasser immer bedeutsamer. Häufig ist es erforderlich, Wasser zur Verwendung als Trink- oder Brauchwasser von Verunreinigungen zu befreien bzw. den Gehalt von Verunreinigungen in einer wäßrigen Lösung zu bestimmen.

Einerseits kann das Entfernen von problematischen Substanzen aus wäßriger Lösung durch eine Reihe von verschiedenen physiko-chemischen Methoden erreicht werden. Als Beispiele wären hier Filtration, Austauschchromatographie und/oder Umkehrosmose zu nennen. Bei diesen Verfahren werden jedoch die problematischen Substanzen nicht abgebaut, sondern sie konzentrieren sich in einem abgesonderten Bereich des Gesamtsystems. Nach der Abtrennung werden diese Substanzen dann in hoher Konzentration wieder an die Umwelt abgegeben.

Andererseits können die problematischen Substanzen auch durch biologische Methoden, d.h. durch enzymatischen Abbau in lebenden Bakterienzellen entfernt werden. Dieser Abbau in Zellen beinhaltet jedoch auch eine Reihe von Schwierigkeiten. So treten Probleme bei einer Immobilisation von lebenden Bakterienzellen auf, die metabolischen Nebenprodukte von Bakterien kontaminieren das gereinigte Wasser. In lebenden Systemen sind ferner lange Reaktionszeiten aufgrund der Induktionszeiten und der großen Diffusionsbarrieren, die sowohl das Substrat als auch das Produkt zu überwinden haben (z. B. die Immobilisierungsmatrix und die Zellmembran) erforderlich. Schließlich können auch die Zellen durch die problematischen Substanzen angegriffen und zerstört werden.

Aufgabe der vorliegenden Erfindung war es daher, ein Verfahren zur Wasserreinigung bereitzustellen, bei dem die Nachteile des Standes der Technik zumindestens teilweise beseitigt sind.

Die erfindungsgemäße Aufgabe wird gelöst durch ein Verfahren zur Umsetzung reduzierbarer oder oxidierbarer Substanzen in wäßriger Lösung, worin man die zu behandelnde wäßrige Lösung mit immobilisierten Oxidoreduktasen, gegebenenfalls in Gegenwart von koimmobilisierten Elektronenüberträgern kontaktiert und gleichzeitig Reduktions- oder Oxidationsäquivalente zuführt die man durch Elektrolyse von Wasser erzeugt.

Der Begriff "Umsetzung" im Sinne der vorliegenden Erfindung beinhaltet sowohl eine teilweise Entfernung von reduzierbaren oder oxidierbaren Substanzen zur Wasseranalytik als auch eine vollständige Entfernung zur Wasserreinigung. Dabei unterscheidet sich die wasseranalytische Bestimmung von Substanzen von der vollständigen Entfernung im wesentlichen dadurch, daß aus einer Probe nur eine zum Nachweis einer bestimmten Substanz ausreichende Menge umgesetzt wird.

Durch Verwendung von immobilisierten Enzymen anstelle von lebenden Zellen entfallen alle oben genannten Probleme, die bei der Verwendung von lebenden Mikroorganismen zur Wasserreinigung auftreten. Weiterhin werden die problematischen Substanzen nicht wie bei den physiko-chemischen Reinigungsverfahren in einem Teilbereich des Systems angereichert, sondern direkt in eine umweltverträgliche Form umgewandelt.

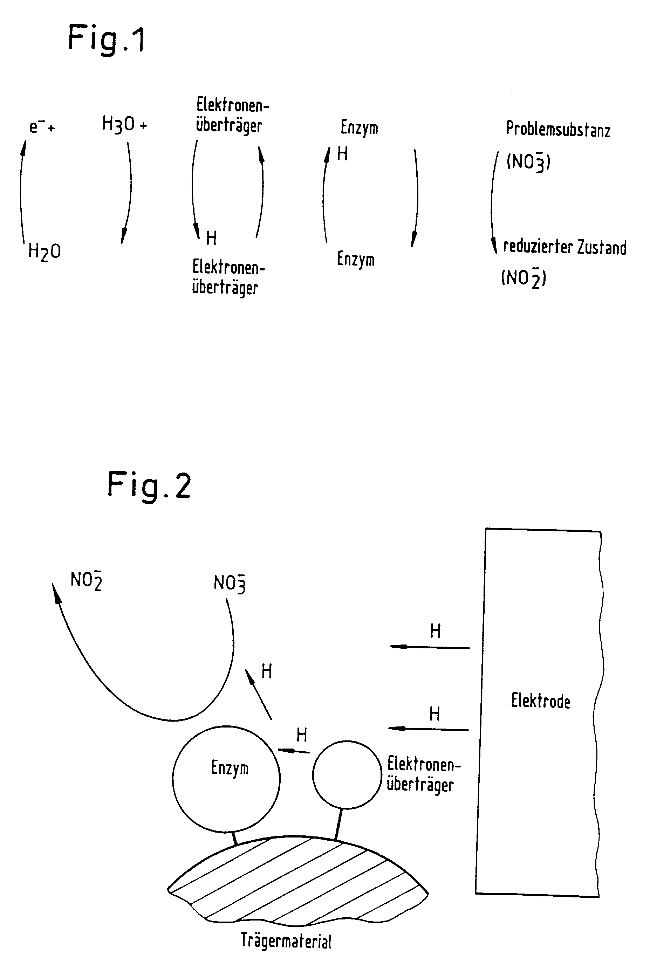

Durch das erfindungsgemäße Verfahren werden die Oxidations- bzw. Reduktionsäquivalente auf die Problemsubstanzen mittels immobilisierter Biokatalysatoren (Enzyme der Oxidoreduktasegruppe) und gegebenenfalls zusätzlich koimmobilisierter Elektronenüberträger übertragen. Verwendet man das erfindungsgemäße Verfahren zur Umsetzung einer reduzierbaren Substanz in wäßriger Lösung, so werden Elektronen auf die zu entfernende Substanz übertragen, verwendet man das erfindungsgemäße Verfahren zur Umsetzung einer oxidierbaren Substanz, so überträgt man dagegen Elektronen von der zu entfernenden Substanz auf ein Oxidationsmittel. Figur 1 zeigt schematisch den Ablauf einer Reduktion am Beispiel der Reduktion von Nitrat zu Nitrit. Der erste Teilschritt dieser Reaktion ist die Übertragung von Reduktions- äquivalenten (hier elektrolytisch erzeugter atomarer Wasser-stoff) auf den Elektronenüberträger. Von dort wird in einem zweiten Teilschritt das Reduktionsäquivalent auf das Enzym (Nitratreduktase) und von dort schließlich auf das Substrat (Nitrat) übertragen, das auf diese Weise zu Nitrit reduziert wird. Diese drei Teilschritte laufen an sich voneinander unabhängig ab. Durch das erfindungsgemäße Verfahren werden diese drei Reaktionen zu einem Gesamtsystem verknüpft (siehe Figur 2).

Das erfindungsgemäße Verfahren dient insbesondere zur Entfernung von Problemsubstanzen aus Trinkwasser oder Abwässern bzw. zur Analyse von Trinkwasser oder Abwässern. Durch das erfindungsgemäße Verfahren lassen sich Substanzen umsetzen, die aus der Gruppe, bestehend aus

(a) anorganischen Salzen und

(b) organischen Verbindungen

ausgewählt sind. Als anorganische Salze kann man beispielsweise Nitrat, Sulfat, Cyanid, Sulfid (bzw. $H_2S$) und Phosphat entfernen. Besonders bevorzugt ist die Umsetzung, d.h. die Entfernung oder/und Bestimmung von Nitrat. Als organische Substanzen, die in wäßriger Lösung umgesetzt werden können, wären insbesondere

2

Herbizide, Pestizide, Öl, Phenole, Lösungsmittel, Fette, Detergenzien (Tenside, Waschmittel) zu nennen. Dies bedeutet, daß man aliphatische oder aromatische Kohlenwasserstoffe, halogenierte Kohlenwasserstoffe (z.B. Dioxine), phosphororganische Verbindungen, Alkohole, Ketone, Phenole oder ähnliche Verbindungen durch das erfindungsgemäße Verfahren in wäßriger Lösung umsetzen kann. Besonders bevorzugt ist die Entfernung oder/und Bestimmung von Alkoholen, insbesondere Methanol (durch Oxidation) oder von halogenierten Kohlenwasserstoffen (durch reduktive Dehalogenierung).

Neben der vollständigen Entfernung von reduzierbaren und oxidierbaren Substanzen aus wäßriger Lösung ist mit Hilfe des erfindungsgemäßen Verfahrens auch eine analytische Bestimmung von Substanzen möglich. Dazu kann man beispielsweise nur einen Teil eines Wasserstroms mit der immobilisierten Oxidoreduktase, gegebenenfalls in Gegenwart von koimmobilisierten Elektronenüberträgern kontaktieren und gleichzeitig Reduktions- oder Oxidationsäquivalente zuführen. Der Verbrauch der zugeführten Reduktions- bzw. Oxidationsäquivalente ist direkt proportional zur Konzentration einer zu bestimmenden Substanz im Wasserstrom. Die Ermittlung dieses Verbrauchs und damit die Messung der Konzentration der zu bestimmenden Substanz kann dann wiederum nach an sich bekannten Verfahren, z.B. potentiometrische oder amperometrische Messung erfolgen.

Die Immobilisierung von Oxidoreduktasen und gegebenenfalls Elektronenüberträgern findet auf einem festen Trägermaterial statt. Geeignete Trägermaterialien sind dem Fachmann bekannt. Es kommen beispielsweise Kunststoffe (Nylon, Polyacrylamid, Polymethylmetacrylat, Polystyrol etc.), Polysaccharide (Agarose, Dextran etc.), Silicate (Sand, Kieselgel, Glas, Keramik etc.) sowie andere Feststoffe (Silicium, Graphit), also die häufig für die Enyzmfixierung bzw. -immobilisierung verwendeten Materialien in Betracht. Ist die Anwesenheit von koimmobilisierten Elektronenüberträgern erforderlich, so befinden sich diese vorzugsweise auf dem selben Trägermaterial wie das Enzym, d.h. Oxidoreduktasen und Elektronenüberträger sind in unmittelbarer molekularer Nachbarschaft auf dem festen Trägermaterial immobilisiert. Andererseits kann der Elektronenüberträger auch direkt an das immobilisierte Enzym bzw. das Enzym direkt an den immobilisierten Elektronenüberträger gebunden sein.

Die dreidimensionale makroskopische Struktur des Trägermaterials kann beliebig gewählt werden. Sie kann zylinderförmig (Stäbchen, Fäden), eben (Folien), kugelförmig oder netzförmig sein. Vorzugsweise verwendet man das Trägermaterial in feinverteilter Form, d.h. kugelförmig und porös mit einem Kugeldurchmesser von 0,5 bis 120 µm, besonders bevorzugt von 1 bis 60 µm.

Die Enzyme und die Elektronenüberträger können an die Matrizen entweder direkt, durch Quervernetzung oder über chemische Verbindungsarme (Spacer), mit oder ohne vorherige Behandlung der Enzyme oder des Trägermaterials gebunden werden. Derartige Methoden sind dem Fachmann bekannt. Beispielsweise ist eine Immobilisierung durch eine Epoxy-, Cyanbromid-, Diisocyanat-, Sulfonylchlorid-, Carbodiimid- oder 2-Fluor-1-methylpyridiniumtoluol-4-sulfonat (FMP), Glutaraldehyd, EDC oder eine andere Verbindung möglich.

Die Aktivierung eines Trägermaterials durch Cyanbromid ist z.B. bei Kohn und Wilcheck (Biochem.Biophys.Res.Comm. 107 (1982), 878-884) beschrieben. Die Aktivierung von Trägermaterial durch organische Sulfonylchloride ist ebenfalls bekannt (Nilsson und Mosbach, Biochem.Biophys.Res.Comm. 102 (1981), 449-457). Eine Methode zur Aktivierung von Hydroxylgruppen einer Trägermatrix durch Reaktion mit TMP in Gegenwart von Triethylamin ist bei Ngo, Bio/Technology Vol. 4 (1986), 134-137) beschrieben. Dabei wird der Ligand (im Falle der vorliegenden Erfindung eine Oxidoreduktase oder ein Elektronenüberträger) über Amino- oder Thiolgruppen an die aktivierte Matrix gebunden. Weiterhin ist auch die Aktivierung von Trägermaterial durch Carbonylierung z.B. mit 1,1'-Carbonyldiimidazol bekannt (Hearn, Meth.Enzymol. 135 (1987), 102-113). Zur Immobilisierung von Oxidoreduktasen und Elektronenüberträgern kann die (aktivierte oder nicht aktivierte) Trägermatrix direkt mit der Oxidoreduktase oder dem Elektronenüberträger oder zunächst mit einem bifunktionellen Spacermolekül umgesetzt werden, an das in einem zweiten Schritt die Oxidoreduktase oder der Elektronenüberträger gekoppelt wird. Als bevorzugte Fixierungsmethoden verwendet man die Diisocyanat- (Biebricher und Luce, U.S. Patent 4.177.038), die Glutardialdehyd- (Korn et al., J. Mol. Biol. 65 (1972), 525-529) und die EDC-Methode (Yamada et al., Biochemistry 20 (1981), 4836-4842).

Zur Umsetzung von Problemstoffen in wäßriger Lösung verwendet man erfindungsgemäß immobilisierte Oxidoreduktasen. Will man eine Substanz durch Oxidation in wäßriger Lösung umsetzen, so verwendet man eine Oxidase, während man zur reduktiven Umsetzung einer Substanz eine Reduktase verwendet. Die Oxidase bzw. Reduktase muß eine enzymatische Spezifität für die im Wasser umzusetzende Substanz besitzen. So verwendet man z.B. für die Oxidation von Methanol eine Alkoholoxidase. Bei einem mehrstufigen Oxidations- oder Reduktionsprozeß kann man mehrere Enzyme gleichzeitig oder hintereinandergeschaltet verwenden. Beispielsweise sind für die Reduktion von Nitrat zu Nitrit eine Nitratreduktase, für die Reduktion von Nitrit zu $N_2O$ eine Nitritreduktase und für die Reduktion von $N_2O$ zu $N_2$ eine $N_2O$-Reduktase erforderlich. Für die reduktive Dehalogenierung von halogenierten organischen Verbindungen kann man eine oder mehrere Dehalogenasen verwenden.

Die Nitratreduktase kann aus dem Pilz Aspergillus oder aus höheren Pflanzen (Campbell in: Molecular and Genetic Aspects of Nitrate Assimilation, S. 125-154, herausgegeben von Wray und Kinghorn, Oxford Univ. Press 1989) z.B. Mais (Zea mays) gewonnen werden. Nitrit- und $N_2O$-Reduktase sind als Enzymgemisch aus Rhodopseudomonas sphaeroides f.sp. denitrificans erhältlich (Michalski und Nickolas, Biochim.Biophys.Acta 828 (1985), 130-137; Michalski et al., Biochim.Biophys.Acta 872 (1986), 50-60). Dehalogenasen können z.B. aus in Sedimenten vorkommenden Mikroorganismen (Suflita et al., Science 218 (1982), 1115-1117), aus Xanthobacter autotrophicus (Janssen et al., Applied and Environmental Microbiology 49 (1985), 673-677), Methan-oxydierenden Bakterien (Little et al., Applied and Environmental Microbiology 54 (1988), 951-956) oder aus Arthrobacter sp. (Scholtz et al., Applied and Evironmental Microbiology 54 (1988), 3034-3038) gewonnen werden. Zum oxidativen Abbau von Methanol kann z.B. Alkoholoxidase (EC 1.1.3.13) aus Hefe (Saccharomyces) verwendet werden. Bei der Reaktion entsteht Formaldehyd, das dann in zwei weiteren Schritten durch Aldehyd-Dehydrogenase zu Formiat und dann durch Formiat-Dehydrogenase zu $CO_2$ und $H_2O$ abgebaut wird. Bei dem erfindungsgemäßen Verfahren können jedoch auch andere Oxidoreduktasen, die für den jeweils gewünschten Zweck geeignet sind, verwendet werden. Die betreffenden Enzyme werden dazu an ein unlösliches Trägermaterial z.B. über chemische Brücken kovalent gebunden. Je nach Bindung und Trägermaterial und Enzym zeigen die immobilisierten Enzyme 40 bis über 100 % der Ausgangsaktivität in freier Lösung.

Bei einer Oxidation werden der zu oxidierenden Substanz Elektronen entzogen, während bei einer Reduktion Elektronen auf die zu reduzierende Substanz übertragen werden. Dafür sind in der Regel Elektronenüberträger erforderlich. In physiologischen Systemen bei freien Enzymen ist der Elektronenüberträger häufig $NAD^+$, das zu NADH reduziert werden kann. Bei den immobilisierten Systemen kann NAD diese Aufgabe in der Regel nicht erfüllen. Stattdessen kann eine Reihe anderer künstlicher Elektronenüberträger benutzt werden. Im allgemeinen sind dies Farbstoffe. Als Farbstoffe geeignet haben sich z.B. Bromphenol Blau, Vertreter der Viologengruppe (Bipyridium-Farbstoffe), Methylen Blau, Phenazin oder Resazurin erwiesen. Als besonders geeignet haben sich Azur A, Bromphenol Blau, Cibacron Blau, Neutral Rot und Safranin T erwiesen. Zur Untersuchung wurden die ausgewählten Farbstoffe an eine Matrix gebunden. Dabei wurde die Belegungsdichte so gewählt, daß in etwa jede zweite Bindungsstelle der Matrix durch ein Farbstoffmolekül belegt ist. An die dazwischenliegenden Bindungsstellen wurde die jeweils verwendete Oxidoreduktase gekoppelt. Dabei wurde festgestellt, daß durch die Immobilisierung die Eigenschaften des Gesamtsystems zum Teil verändert wurden. Nicht jeder Farbstoff, der für eine freie Oxidoreduktase als Elektronenüberträger wirken kann, ist auch in der Lage, diese Funktion auch für das immobilisierte Enzym zu erfüllen.

Es wurde auch gefunden, daß sich als Elektronenüberträger, die für das erfindungsgemäße Verfahren mit der Oxidoreduktase koimmobilisiert sind, auch ungiftige Lebensmittelfarbstoffe eignen. Beispiele für Lebensmittelfarbstoffe, die in immobilisiertem Zustand als Elektronenüberträ-ger wirken können, sind etwa Curcumin und Derivate davon, Chinolinfarbstoffe (z.B. Chinolingelb) und Patentblau-Farbstoffe (z.B. Patentblau V). Ein großer Vorteil bei der Verwendung von Lebensmittelfarbstoffen als Elektronenüberträger für das erfindungsgemäße Verfahren beruht auf ihrer geringen Toxizität. Dies ist insbesondere bei solchen Ausführungsformen bedeutsam, in denen eine wäßrige Lösung von Schadstoffen gereinigt wird und anschließend als Trinkwasser oder/und zur Herstellung von Getränken eingesetzt wird. Ein weiterer Vorteil von einigen Lebensmittelfarbstoffen ist ihr geringer Preis, so ist z.B. Curcumin erheblich billiger als Methylviologen.

Bei der reduktiven Umsetzung von Nitrat in wäßriger Lösung haben sich etwa folgende Kombinationen als besonders geeignet erwiesen:
Nitratreduktase aus Mais koimmobilisiert mit Azur A oder Bromphenol Blau, das Enzymgemisch Nitrit- und $N_2O$-Reduktase koimmobilisiert mit Neutral Rot oder Safranin T.

Weiterhin als besonders geeignet haben sich Kombinationen von Nitratreduktase aus Mais mit den koimmobilisierten Lebensmittelfarbstoffen Curcumin oder Patentblau V, sowie von Nitritreduktase mit den koimmobilisierten Lebensmittelfarbstoffen Curcumin, Patentblau V oder Chinolingelb erwiesen.

Für andere Systeme können sich jedoch auch natürliche Elektronenüberträger als geeignet erweisen, insbesondere solche Elektronenüberträger, die an membranstämmigen Enzymkomplexen beteiligt sind. Beispiele für natürliche Elektronenüberträger sind: Ascorbat, Eisenproteine (Flavo-Proteine, Ferridoxine, Rubidoxin, Zytochrome), Flavine (FAD, FMN), Pyridinnukleotide (NAD, NADP), Pterine, Pteridine und Chinone (z.B. Ubichinon).

Verwendet man als Oxidoreduktase ein Protein, das den elektronenübertragenden Kofaktor bereits enthält, ist das Koimmobilisieren eines Elektronenüberträgers nicht erforderlich. Ein Beispiel für ein solches Protein ist die Alkoholoxidase, bei der es sich um ein Flavoprotein handelt.

Da es sich bei dem erfindungsgemäßen Verfahren um eine Reduktion bzw. Oxidation handelt, müssen Reduktions- bzw. Oxidationsäquivalente bereitgestellt werden. Dies könnte prinzipiell durch Zugabe geeigneter chemischer Substanzen geschehen. So könnten man z.B. der zu reinigenden wäßrigen Lösung als Reduk-

tionsmittel Dithionit ($S_2O_4^{2-}$) oder Wasserstoff mit einem geeigneten Katalysator (zur Zerlegung von molekularem Wasserstoff in atomaren Wasserstoff) verwendet werden. Als Oxidationsmittel wären z.B. $H_2O_2$ oder in der wäßrigen Lösung bereits vorhandener Sauerstoff geeignet. Bei dieser chemischen Redoxreaktion können jedoch Probleme wegen der Giftigkeit ($S_2O_4^{2-}$, $H_2O_2$) der verwendeten Substanzen und auch aus Kostengründen Probleme insbesondere bei der Reinigung von Trinkwasser entstehen.

Erfindungsgemäß erzeugt man daher regenerierbare Reduktions- oder Oxidationsäquivalente auf elektrochemischem Weg, insbesondere durch Elektrolyse von Wasser. Die elektrolytische Spaltung von Wasser erzeugt Hydroxydionen ($OH^-$) und Hydroniumionen ($H_3O^+$). Die positiv geladenen Hydroniumionen wandern zur Kathode (Minus-Pol), wo sie ein Elektron aufnehmen und in Wasser und atomaren Wasserstoff zerfallen. Dieser atomare Wasserstoff ist instabil und verbindet sich schnell zu $H_2$-Gas. Entsprechend wandern die negativ geladenen Hydroxydionen zur Anode (Plus-Pol), wo sie ein Elektron abgeben und in Wasser und atomaren Sauerstoff zerfallen. Bei Anwesenheit von Oxidasen bzw. Reduktasen sowie gegebenenfalls Elektronenüberträgern in Nachbarschaft der Kathode erfolgt jedoch eine schnelle Oxidation bzw. Reduktion von für die jeweilige Oxidoreduktase spezifischen Substraten. So werden z.B. die immobilisierten Farbstoffe von dem nur kurzlebigen atomaren Wasserstoff an der Kathode sehr schnell reduziert, wobei die Reduktion proportional zur Potentialdifferenz ist (bis zu 70 Volt). Die Effektivität der Elektronenübertragung hängt dabei von der Größe der Oberfläche der Kathode ab.

In ungepufferten Systemen werden bei einer elektrochemischen Redoxreaktion die Protonen- und Hydroxydionenkonzentrationen innerhalb von 1 bis 2 Minuten bereits recht groß. Dies würde die Aktivität eines ungepufferten Systems (aufgrund der pH-Wert-Änderung) stark begrenzen, da bei der Reinigung von Wasser, insbesondere Trinkwasser, der Lösung auch kein Puffersalz zugeführt werden sollte. Man führt daher das erfindungsgemäße Verfahren vorzugsweise in einer potentiometrischen Zelle durch, welche die immobilisierten Oxidoreduktasen sowie gegebenenfalls die koimmobilisierten Elektronenüberträger enthält, indem man bei einer geeigneten Spannung Reduktions- oder Oxidationsäquivalente erzeugt und die Zelle kontinuierlich mit der zu reinigenden Lösung durchströmt. Dabei hat sich die Einstellung einer Verweilzeit der Lösung in der Zelle von 0,2 bis 5 Zellvolumina/Minute, vorzugsweise 0,5 bis 2 Zellvolumina/Minute als günstig erwiesen. Dabei werden die immobilisierten Oxidoreduktasen sowie gegebenenfalls die Elektronenüberträger in der Zelle zurückgehalten, während die überschüssigen Ionen aus der Zelle gewaschen werden. Vorzugsweise verwendet man ein System mit getrennter Anode und Kathode. Die notwendige elektrolytische Verbindung kann z.B. über einen groben Glasfilter ermöglicht werden. Die Eluate beider Elektroden können nach der Reaktion wieder zusammengeführt werden. Dabei wird nach Durchfluß durch das Gesamtsystem wieder ein neutraler pH-Wert erreicht.

Es mag jedoch Anwendungsbereiche geben, wo ein Zusatz von Puffersubstanzen zur wäßrigen Lösung, die gereinigt werden soll, akzeptiert werden kann. In diesem Fall wäre dann eine Durchflußzelle keinesfalls obligat.

Sind für die Umsetzung einer Substanz in wäßriger Lösung mehrere Reduktions- bzw. Oxidationsschritte erforderlich (z.B. bei der Reduktion von Nitrat), müssen in aller Regel mehrere verschiedene Enzyme eingesetzt werden, die gegebenenfalls auch mit verschiedenen Elektronenüberträgern koimmobilisiert sind. Daher kann z.B. eine Zelle auf jeweils verschiedenen Trägern unterschiedliche Enzym/Elektronenüberträger-Systeme enthalten. Es ist jedoch auch möglich, mehrere Zellen in Serie zu schalten, wobei dann in einer Zelle z.B. nur ein Enzym/Elektronenüberträger-System vorliegt. Die letztgenannte Methode ist bevorzugt.

Die Konzentration der Problemsubstanz in der zu reinigenden bzw. zu analysierenden wäßrigen Lösung kann einen weiten Bereich umfassen. Entsprechend dieser Konzentration lassen sich nämlich auch die Bedingungen des erfindungsgemäßen Verfahrens variieren. Bei geringer Konzentration der Problemsubstanzen sind entsprechend kleinere Zellenanordnungen (d.h. geringe Enzym- und ggf. Elektronenüberträgermengen oder/und geringe Mengen an Redoxäquivalenten) oder/und bei einer Durchflußzelle höhere Durchflußgeschwindigkeiten möglich. Bei einer höheren Konzentration der Problemsubstanzen sind dementsprechend größere Zellenanordnungen oder/und geringe Durchflußgeschwindigkeiten erforderlich. Überdies können zu hohe Konzentrationen der Problemsubstanzen durch Verdünnen mit Wasser auf geeignete Werte herabgesetzt werden. Vorzugsweise liegt der Konzentrationsbereich für die Anwendung des erfindungsgemäßen Verfahrens von 0,01 mmol/l bis etwa 10 mmol/l, wobei aber auch darüber und darunter liegende Werte nicht ausgeschlossen sind.

Die Potentialdifferenz in der Elektrolysezelle muß sich nach den verwendeten Oxidoreduktasen richten. So zeigt sich bei der Nitratreduktion, daß bei einer Spannung von über 4 V die Reduktasen innerhalb einer gewissen Zeitspanne inaktiviert werden. Dagegen kann die Oxidation von Methanol in Gegenwart von Alkoholoxidase bei einer Spannung von über 10 V durchgeführt werden.

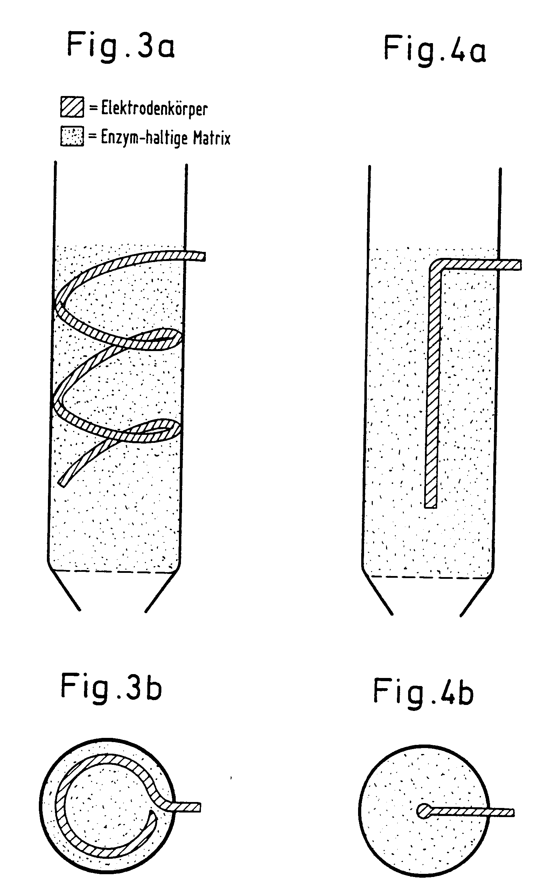

Weiterhin ein Gegenstand der vorliegenden Erfindung ist eine potentiometrische Zelle zur Umsetzung reduzierbarer oder oxidierbarer Substanzen in wäßriger Lösung, die einen Elektrodenkörper und immobilisierte Oxidoreduktasen sowie gegebenenfalls koimmobilisierte Elektronenüberträger enthält. Dabei kann die Form des Elektrodenkörpers an sich beliebig sein. Er kann fadenförmig oder als Band ausgebildet sein (spiralförmig, linear), eine spiralförmig gewickelte Folie sein, es kann sich auch um eine massive Elektrode mit Bohrungen, Anordnungen aus gestapelten Fritten oder gewobenen Netzen handeln. Bevorzugte Ausführungsformen des erfindungsgemäßen Elektrodenkörpers sind aus den Figuren 3 bis 8 ersichtlich.

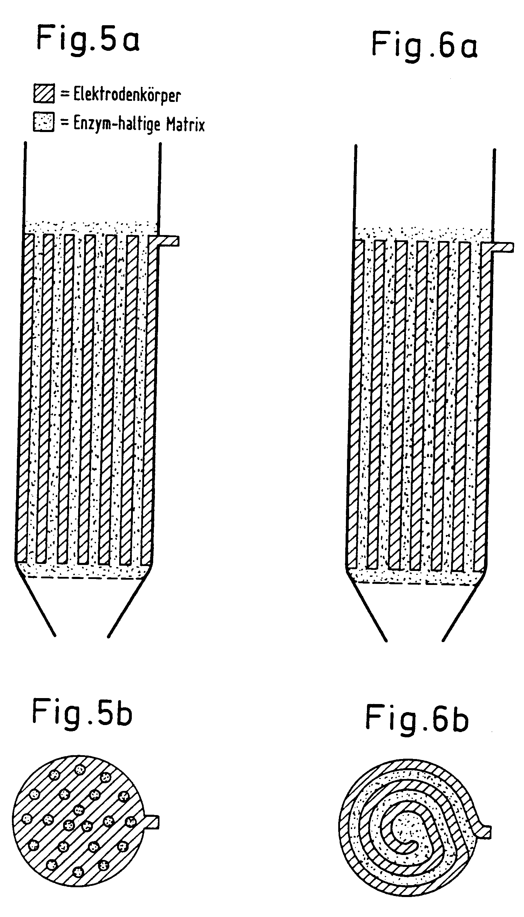

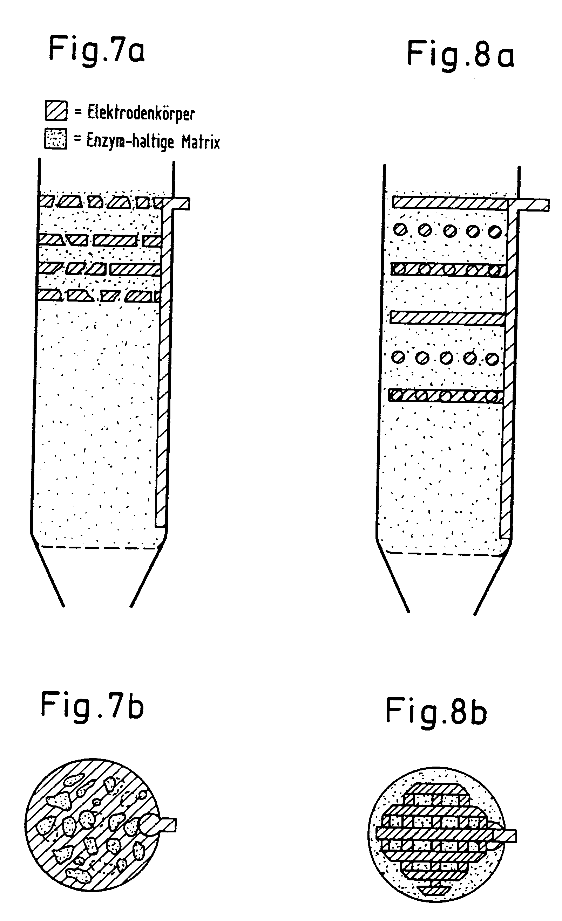

Fig. 3 und 4 zeigen draht- bzw. bandförmig ausgebildete Elektroden, die sich spiralförmig (Fig. 3) oder gerade (Fig. 4) durch eine aus feinkörnigem Trägermaterial bestehende Enzymhaltige Matrix erstrecken. Fig. 5 und 6 zeigen einteilige Elektroden, die in eine Enzym-haltige Matrix eingebettet sind, wobei die Elektrode entweder als gelöchertes Klötzchen (Fig. 5) oder als spiralförmig gewickelte Folie (Fig. 6) ausgebildet ist. Fig. 7 und 8 zeigen mehrteilige Elektroden, die als übereinander gestapelte Fritten (Fig. 7) oder Netze (Fig. 8) ausgebildet sein können.

Oxidoreduktasen und die gegebenenfalls vorhandenen Elektronen überträger in der erfindungsgemäßen Zelle sind z.B. direkt, durch Quervernetzung oder über einen Spacer mittels Epoxy-, Cyanbromid-, Diisocyanat-, Carbodiimid- Glutardialdehyd-, EDC- oder FMP-Verbindung auf dem Trägermaterial immobilisiert. Verwendet man als Elektrodenkörper ein Halbleitermaterial wie z.B. Silicium oder Graphit, können die Oxidoreduktasen und gegebenenfalls Elektronenüberträger direkt auf der Oberfläche des Elektrodenkörpers immobilisiert sein.

Alternativ dazu können als Trägermaterial Polymere aus natürlichem Material oder Kunststoffen benutzt werden, die mehrere chemisch unterschiedliche Bindestellen in vorgegebenem Abstand bieten (z. B. bifunktionell sind), so daß Enzyme und Elektronenüberträger alternierend direkt nebeneinander in molekularer Nachbarschaft gebunden werden können. Es wird angestrebt, daß diese Polymere Strom leiten können. Bei einer direkten Immobilisierung von Oxidoreduktase und gegebenenfalls Elektronenüberträger auf der Elektrode können Reduktions- oder Oxidationsäquivalente direkt vom Elektrodenkörper auf die Oxidoreduktasen oder gegebenenfalls auf die Elektronenüberträger übertragen werden, ohne daß eine Elektrolyse des Wassers notwendig ist (d. h. die Reaktion könnte unterhalb des Wasserpotentials von 1,28 V stattfinden).

Ansonsten erfolgt die Immobilisierung auf einem geeigneten Trägermaterial oder einer Mischung von geeigneten Trägermaterialien (siehe oben), die nicht Bestandteil des Elektrodenkörpers sind.

Vorzugsweise ist dabei das feste Trägermaterial in feinteiliger Form ausgebildet, so daß der Elektrodenkörper in das Trägermaterial eingebettet werden kann (siehe z.B. die Figuren 3 bis 8). Man kann auch als Elektrodenkörper eine Art Metallschaum verwenden, in dessen Poren oder Löchern sich das feinteilige Trägermaterial befindet, das die Enzyme und gegebenenfalls Elektronenüberträger in immobilisierter Form enthält. Ein derartiges schaumartiges Material kann z.B. aus einem Paket von Lochfolien oder Metallnetzen bestehen, wobei dann die Trägerpartikel in den Poren oder Löchern der Netze bzw. Folien angeordnet wären.

Vorzugsweise ist die erfindungsgemäße Zelle als Durchflußzelle ausgebildet. Dazu kann die Zelle z.B. an zwei gegenüberliegenden Seiten durch Glasfritten begrenzt sein. Der von der Zelle gebildete Raum wird dann von der zu reinigenden wäßrigen Flüssigkeit mit einer einstellbaren Geschwindigkeit durchströmt, so daß eine pH-Verschiebung vermieden wird, die eine Inaktivierung der Enzyme bewirken könnte.

Weiterhin betrifft die vorliegende Erfindung die Verwendung der erfindungsgemäßen potentiometrischen Zelle zur Umsetzung von reduzierbaren oder oxidierbaren Substanzen in Trinkwasser oder Abwässern. Insbesondere kann die erfindungsgemäße Zelle zur Umsetzung, d.h. zur Bestimmung und insbesondere zur Entfernung von Nitrat, Methanol oder halogenierten Kohlenwasserstoffen eingesetzt werden.

Die folgenden Beispiele sollen die vorliegende Erfindung in Verbindung mit den Figuren 1 bis 8 weiter verdeutlichen. Es zeigen:

| | |
|---|---|
| Figur 1 | eine schematische Darstellung des Ablaufs einer Reduktion am Beispiel der Reduktion von Nitrat zu Nitrit, |
| Figur 2 | eine erfindungsgemäße Anordnung mit immobilisiertem Enzym und Elektronenüberträger und einer Elektrode zur Reduktion von Nitrat zu Nitrit, |
| Figur 3 | eine potentiometrische Zelle mit einem spiralförmig ausgebildeten Elektrodenkörper, der in eine enzymhaltige Matrix eingebettet ist, wobei Fig. 3a einen Längsschnitt und Fig. 3b einen Querschnitt durch die Zelle darstellt, |
| Figur 4 | eine potentiometrische Zelle mit einem drahtförmigen, geraden Elektrodenkörper, der in eine enzymhaltige Matrix eingebettet ist, |
| Figur 5 | eine potentiometrische Zelle mit einem einteiligen, als gelöchertes Klötzchen ausgebildeten Elektrodenkörper, der in eine enzymhaltige Matrix eingebettet ist, |
| Figur 6 | eine potentiometrische Zelle mit einem einteiligen, als spiralförmige gewickelte Folie ausgebildeten Elektrodenkörper, der in eine enzymhaltige Matrix eingebettet ist, |
| Figur 7 | eine potentiometrische Zelle mit einem mehrteiligen, aus übereinandergestapelten Fritten bestehenden Elektrodenkörper, der in eine enzymhaltige Matrix eingebettet ist, und |

Figur 8       eine potentiometrische Zelle mit einem mehrteiligen, aus übereinandergestapelten Netzen bestehenden Elektrodenkörper, der in eine enzymhaltige Matrix eingebettet ist.

Beispiel 1

Abbau von Nitrat zu Stickstoff

1.1 Auswahl geeigneter Enzyme und Elektronenüberträger

Die Reduktion von Nitrat zu Stickstoff findet in drei Stufen statt. In der ersten Stufe wird Nitrat durch die Nitratreduktase zu Nitrit reduziert. In der zweiten Stufe wird Nitrit durch die Nitritreduktase zu $N_2O$ reduziert und in der dritten Stufe wird $N_2O$ durch die $N_2O$-Reduktase zu $N_2$ reduziert.

Es wurden Nitratreduktasen aus Aspergillus und Mais (Zea mays) sowie ein Gemisch aus Nitrit- und $N_2O$-Reduktasen aus Rhodopseudomonas sphaeroides untersucht. Die Nitratreduktase aus Aspergillus wurde von Boehringer Mannheim bezogen. Immunoaffinitätschromatographisch reine Nitratreduktase aus Mais wurde hergestellt, wie bei Ruoff et al. (1989), Biochem.Biophys.Res.Commun. <u>161</u>, 496-501 beschrieben. Beide Enzyme wurden vor Gebrauch gefriergetrocknet.

Zunächst wurde die Enzymaktivität der <u>freien</u> Enzyme mit <u>immobilisierten</u> Farbstoffen (als Elektronenüberträger) im Vergleich zu den freien Farbstoffen getestet. Dabei wurde die Aktivität des freien Enzyms in Verbindung mit dem freien Farbstoff Methylviologen auf 100 % gesetzt. Als Reduktionsmittel wurde Natriumdithionit benutzt.

Eine Standardreaktion enthielt 2,5 µmol $KNO_3$ in 0,5 ml 50 mmol/ml Na, K-Phosphatpuffer pH 7,2. 1,5 µmol Farbstoff wurden mit 100 m Units Enzym gemischt und die Reaktion in Anwesenheit von 20 µl 10 % Natriumdithionit (in 50 µg/ml $NaHCO_3$) gestartet. Nach 15-minütiger Inkubation bei 25°C wurde das Reaktionsgemisch durch starkes Schütteln reoxidiert und das Nitrit durch die Griess-Ilosvay-Reaktion bestimmt (Kundu und Nikolas (1985), Arch.Microbiol. <u>141</u>, 57-62). Der Ansatz wurde in gut verschließbaren 1,5 ml Reaktionsgefässen durchgeführt. Kontrollen enthielten entweder keinen Farbstoff oder durch Erhitzen inaktiviertes Enzym. 1 Unit Enzym reduziert 1 µmol Nitrat pro Minute in Anwesenheit von 3 µmol/ml Methylviologen. Die Ergebnisse dieses Tests sind in der folgenden Tabelle 1 ersichtlich.

Tabelle 1

| Farbstoff | Nitratreduktase | | Nitritreduktase |
|---|---|---|---|
| | Zea | Aspergillus | |
| Azur A | 30,8 | 9,7 | 71,9 |
| Cibacron blau | 38,6 | 48,6 | 91,0 |
| Cresyl violett | 13,0 | 2,0 | 84,9 |
| Nil Blau | 10,0 | 16,5 | 66,5 |
| Neutral Rot | 55,0 | 10,8 | 94,5 |
| Safranin T | 44,5 | 4,1 | 66,2 |
| Thionin | 14,5 | 15,9 | 62,6 |
| Bromphenol Blau | 21,5 | 8,4 | 58,3 |
| Curcumin | 51,4 | NG | 116,4 |
| Patentblau V | 36,8 | NG | 112,0 |
| Chinolingelb | NG | NG | 121,8 |

NG = nicht getestet

Anschließend wurde die Aktivität des <u>immobilisierten</u> Enzyms mit dem angegebenen <u>koimmobilisierten</u>

Farbstoff relativ zur Enzymaktivität des immobilisierten Enzyms in Anwesenheit des freien Farbstoffs Methylviologen bestimmt. Als Reduktionsmittel wurde Natriumdithionit benutzt. Die Ergebnisse sind aus der folgenden Tabelle 2 ersichtlich.

## Tabelle 2

| Reduktasen: | A | B | C |
|---|---|---|---|
| Farbstoffe: | | | |
| Azur A | 97,6 | 33,1 | 62,5 |
| Cibacron Blau | 20,4 | 50,1 | 55,3 |
| Neutral Rot | 16,4 | 8,7 | 102,0 |
| Safranin T | 75,0 | 3,0 | NG |
| Bromphenol Blau | 93,6 | NG | 50,0 |
| Curcumin | 109,7 | NG | 97,3 |
| Patentblau V | 112,2 | NG | 105,2 |
| Chinolingelb | NG | NG | 113,5 |

A = Zea Nitratreduktase

B = Aspergillus Nitratreduktasse

C = Rhodopseudomonas Nitritreduktase

NG = nicht getestet

Aus den Tabellen 1 und 2 wird ersichtlich, daß sich die Eigenschaften des Systems aus Enzym und Farbstoff durch die Immobilisierung erheblich ändern können. Für die Nitratreduktase aus Mais (Zea mays) sind als Farbstoffe Azur A, Bromphenol Blau, Curcumin und Patentblau V besonders geeignet, während für die Nitritreduktase aus Rhodopseudomonas die Farbstoffe Neutral Rot, Curcumin, Patentblau V und Chinolingelb besonders geeignet ist.

1.2 Durchführung des Nitratabbaus

Es wurde eine 1 ml-Reaktorzelle, die koimmobilisierte Nitratreduktase und Azur A enthält, mit einer anderen 1 ml-Reaktorzelle, die immobilisierte Nitrit- und $N_2O$-Reduktase zusammen mit koimmobilisiertem Neutral Rot enthält, verknüpft. Die beiden Reaktorzellen wurden hintereinandergekoppelt. Es wurden zwei verschiedene Substratkonzentrationen untersucht:
a) hohe Konzentration
10 ml einer 10 mmol/l Nitratlösung wurden durch beide Säulen (in Serie geschaltet) mit einer Flußrate von 1 ml/min und 4 V Spannung geleitet. Die im Eluat gemessenen Nitrat- und Nitrit-Konzentrationen sind in Tabelle 3 aufgeführt. Demnach reduzierten die Säulen 1,272 µmol oxidierten Stickstoff/min..
b) niedrige Konzentration
Der obige Versuch wurde mit 10 ml einer 1 mmol/l Nitratlösung wiederholt. Im Eluat war weder Nitrat noch Nitrit nachweisbar. Außerdem konnten kein NO und kein $N_2O$ (nachweisbar als $HNO_2$ nach Reoxidation mit Sauerstoffgas) im Eluat nachgewiesen werden (Tabelle 3).

## Tabelle 3

| | Konzentration (mmol) | | |
|---|---|---|---|
| | $NO_3^-$ | $NO_2^-$ | $NO/N_2O$ |
| **1. hohe $NO_3^-$ Konzentration** | | | |
| (Lösung) | 10,0 | 0 | 0 |
| (Eluat) | 8,69 | 0,38 | 0 |
| **2. niedrige $NO_3^-$ Konzentration** | | | |
| (Lösung) | 1,0 | 0 | 0 |
| (Eluat) | 0 | 0 | 0 |

Beispiel 2

Methanolabbau durch Alkoholoxidase

Alkoholoxidase (EC 1.1.3.13) aus Hefe (Saccharomyces) ist ein Flavoprotein, das bereits einen elektronenübertragenden Kofaktor enthält. Hier ist also eine Koimmobilisation eines Elektronenüberträgers (Farbstoffes) nicht notwendig. Beim Abbau von Methanol entsteht Formaldehyd.

38 Einheiten Alkoholoxidase wurden auf Fractogel HSK immobilisiert und in einer elektrochemischen Reaktorzelle untersucht. Die enzymatische Aktivität in 10 mmol/l Phosphatpuffer mit Methanol als Substrat wurde auf 100 % gesetzt. Vor dem eigentlichen Versuch wurde das Reaktionsgemisch mit Dithionit reduziert, um den Luftsauerstoff zu entfernen. In diesem Zustand wurden nur 13 Einheiten (34 %) Aktivität gemessen. Anschließend wurde die Reaktorzelle so gepolt, daß im Enzymbereich oxidierende Bedingungen herrschten. Bei 12 V und 1 mA in der Zelle wurden 21,9 Einheiten (58 %) Aktivität gemessen.

Dieses Ergebnis zeigt, daß die immobilisierte Alkoholoxidase auch ohne koimmobilisierten Elektronenüberträger offensichtlich in der Lage ist, Elektronen von Substraten aufzunehmen und auf ein elektrolytisch erzeugtes Oxidationsmittel zu übertragen.

## Patentansprüche

1. Verfahren zur Umsetzung reduzierbarer oder oxidierbarer Substanzen in wäßriger Lösung,
   **dadurch gekennzeichnet,**
   daß man die zu behandelnde wäßrige Lösung mit immobilisierten Oxidoreduktasen, gegebenenfalls in Gegenwart von koimmobilisierten Elektronenüberträgern kontaktiert und gleichzeitig Reduktions- oder Oxidationsäquivalente zuführt, die man durch Elektrolyse von Wasser erzeugt.

2. Verfahren nach Anspruch 1,
   **dadurch gekennzeichnet,**
   daß man Oxidoreduktasen und Elektronenüberträger in unmittelbarer molekularer Nachbarschaft oder/und direkt aneinander gebunden auf einem festen Trägermaterial immobilisiert verwendet.

3. Verfahren nach Anspruch 2,
   **dadurch gekennzeichnet,**
   daß man als Elektronenüberträger einen nicht-toxischen Farbstoff auswählt.

4. Verfahren nach einem der Ansprüche 1 bis 3,
   **dadurch gekennzeichnet,**
   daß man elektrolytisch erzeugte regenerierbare Reduktions- oder Oxidationsäquivalente zuführt.

5. Verfahren nach einem der Ansprüche 1 bis 4,

**dadurch gekennzeichnet,**
daß man als Reduktionsäquivalent atomaren Wasserstoff verwendet.

6. Verfahren nach einem der Ansprüche 1 bis 5,
**dadurch gekennzeichnet,**
daß man als Oxidationsäquivalent atomaren Sauerstoff verwendet.

7. Verfahren nach Anspruch 4,
**dadurch gekennzeichnet,**
daß man in einer potentiometrischen Zelle, welche die immobilisierten Oxidoreduktasen sowie gegebenenfalls die koimmobilisierten Elektronenüberträger enthält, bei einer geeigneten Spannung Reduktions- oder Oxidationsäquivalente erzeugt und die Zelle kontinuierlich mit der zu reinigenden Lösung durchströmt.

8. Verfahren nach Anspruch 7,
**dadurch gekennzeichnet,**
daß man eine Verweilzeit der Lösung in der Zelle von 0,2 bis 5 Zellvolumina/min. einstellt.

9. Verfahren nach Anspruch 7 oder 8,
**dadurch gekennzeichnet,**
daß man eine Zelle mit getrennten Kathoden- und Anodenbereichen verwendet und die abströmende Lösung aus beiden Bereichen wieder vereinigt.

10. Verfahren nach einem der Ansprüche 7 bis 9,
**dadurch gekennzeichnet,**
daß man mehrere hintereinandergeschaltete Zellen verwendet.

11. Verfahren nach Anspruch 10,
**dadurch gekennzeichnet,**
daß einzelne Zellen unterschiedliche immobilisierte Oxidoreduktasen enthalten.

12. Verfahren nach einem der vorhergehenden Ansprüche,
**dadurch gekennzeichnet,**
daß man in wäßriger Lösung Substanzen umsetzt, die aus der Gruppe, bestehend aus
(a) anorganischen Salzen, insbesondere Nitrat, Sulfat, Cyanid, Sulfid und Phosphat, und
(b) organischen Verbindungen, insbesondere aliphatische oder aromatische Kohlenwasserstoffe, halogenierte Kohlenwasserstoffe, phosphoorganische Verbindungen, Alkohole, Ketone, Phenole und Detergenzien
ausgewählt sind.

13. Verfahren nach Anspruch 12,
**dadurch gekennzeichnet,**
daß man Nitrat in wäßriger Lösung umsetzt.

14. Verfahren nach Anspruch 12,
**dadurch gekennzeichnet,**
daß man Methanol in wäßriger Lösung umsetzt.

15. Verfahren nach Anspruch 12,
**dadurch gekennzeichnet,**
daß man halogenierte Kohlenwasserstoffe in wäßriger Lösung umsetzt.

16. Verfahren nach einem der vorhergehenden Ansprüche,
**dadurch gekennzeichnet,**
daß die Konzentration der in der wäßrigen Lösung umzusetzenden Substanzen etwa 0,01 mmol/l bis etwa 10 mmol/l beträgt.

17. Potentiometrische Zelle zur Umsetzung reduzierbarer oder oxidierbarer Substanzen in wäßriger Lösung,
**dadurch gekennzeichnet,**
daß sie einen Elektrodenkörper und immobilisierte Oxidoreduktasen sowie gegebenenfalls koimmobilisierte Elektronenüberträger enthält.

18. Zelle nach Anspruch 17,
**dadurch gekennzeichnet,**
daß Oxidoreduktasen und gegebenenfalls Elektronenüberträger direkt, durch Quervernetzung oder über einen Spacer mittels Epoxy-, Cyanbromid-, Diisocyanat-, Carbodiimid-, Glutardialdehyd, EDC-, FMP- oder andere Verbindungen immobilisiert sind.

19. Zelle nach Anspruch 17 oder 18,
**dadurch gekennzeichnet,**
daß Oxidoreduktasen und gegebenenfalls Elektronenüberträger auf der Oberfläche eines Trägermaterials immobilisiert sind.

20. Zelle nach Anspruch 19,
**dadurch gekennzeichnet,**
daß das feste Trägermaterial aus Kunststoffen, Polysacchariden, Silikaten, Silicium, Graphit sowie Derivaten und Gemischen davon besteht.

21. Zelle nach Anspruch 19 oder 20,
**dadurch gekennzeichnet,**
daß das feste Trägermaterial in feinteiliger Form ausgebildet ist.

22. Zelle nach Anspruch 21,
**dadurch gekennzeichnet,**
daß der Elektrodenkörper im Trägermaterial eingebettet vorliegt.

23. Zelle nach Anspruch 21,
**dadurch gekennzeichnet,**
daß sich das Trägermaterial in Poren oder Löchern des Elektrodenkörpers befindet.

24. Zelle nach Anspruch 17 oder 18,
**dadurch gekennzeichnet,**
daß Oxidoreduktasen sowie gegebenenfalls Elektronenüberträger auf der Oberfläche des Elektrodenkörpers immobilisiert sind.

25. Zelle nach einem der Ansprüche 17 bis 24,
**dadurch gekennzeichnet,**
daß sie eine Durchflußzelle ist.

26. Zelle nach einem der Ansprüche 17 bis 25,
**dadurch gekennzeichnet,**
daß sie nicht-toxische Farbstoffe, insbesondere Lebensmittelfarbstoffe als Elektronenüberträger enthält.

27. Verwendung einer potentiometrischen Zelle nach einem der Ansprüche 17 bis 25 zur Umsetzung von reduzierbaren oder oxidierbaren Substanzen in Trinkwasser oder Abwässern.

28. Verwendung nach Anspruch 27 zur Umsetzung von Nitrat.

29. Verwendung nach Anspruch 27 zur Umsetzung von Methanol.

30. Verwendung nach Anspruch 27 zur Umsetzung von halogenierten Kohlenwasserstoffen.

**Claims**

1. Process for the conversion of reducible or oxidizable substances in aqueous solution,
**wherein**
the aqueous solution to be treated is contacted with immobilized oxidoreductases, if desired, in the presence of co-immobilized electron carriers and reduction or oxidation equivalents that are produced by electrolysis of water are supplied concurrently.

2. Process as claimed in claim 1,
**wherein**
oxidoreductases and electron carriers are used which are immobilized on a solid carrier material and are in direct molecular proximity or/and bound directly together.

3. Process as claimed in claim 2,
**wherein**
a non-toxic dye is selected as the electron carrier.

4. Process as claimed in one of the claims 1 to 3,
**wherein**
one supplies reduction or oxidation equivalents produced by electrolysis which can be regenerated.

5. Process as claimed in one of the claims 1 to 4,
**wherein**
atomic hydrogen is used as the reduction equivalent.

6. Process as claimed in one of the claims 1 to 5,
**wherein**
atomic oxygen is used as the oxidation equivalent.

7. Process as claimed in claim 4,
**wherein**
reduction or oxidation equivalents are generated at a suitable voltage in a potentiometric cell which contains the immobilized oxidoreductases as well as, if desired, the co-immobilized electron carriers and the solution to be purified is passed continuously through the cell.

8. Process as claimed in claim 7,
**wherein**
the residence time of the solution in the cell is set to 0.2 to 5 cell volumes/min.

9. Process as claimed in claim 7 or 8,
**wherein**
a cell with separate cathode and anode regions is used and the solutions which flow from both regions are combined again.

10. Process as claimed in one of the claims 7 to 9,
**wherein**
several cells connected in series are used.

11. Process as claimed in claim 10,
**wherein**
individual cells contain different immobilized oxidoreductases.

12. Process as claimed in one of the previous claims,
**wherein**
substances are converted in aqueous solution which are selected from the group comprising
(a) inorganic salts, in particular nitrate, sulfate, cyanide, sulfide and phosphate, and
(b) organic compounds, in particular aliphatic or aromatic hydrocarbons, halogenated hydrocarbons, organophosphorus compounds, alcohols, ketones, phenols and detergents.

13. Process as claimed in claim 12,
**wherein**
nitrate is converted in aqueous solution.

14. Process as claimed in claim 12,
**wherein**
methanol is converted in aqueous solution.

15. Process as claimed in claim 12,

**wherein**
halogenated hydrocarbons are converted in aqueous solution.

16. Process as claimed in one of the previous claims,
**wherein**
the concentration of the substances to be converted in the aqueous solution is about 0.01 mmol/l to about 10 mmol/l.

17. Potentiometric cell for converting reducible or oxidizable substances in aqueous solution,
**wherein**
it contains an electrode body and immobilized oxidoreductases as well as, if desired, co-immobilized electron carriers.

18. Cell as claimed in claim 17,
**wherein**
oxidoreductases and, if desired, electron carriers are directly immobilized by cross-linking or via a spacer by means of epoxy, cyanogen bromide, diisocyanate, carbodiimide, glutardialdehyde, EDC, FMP or other compounds.

19. Cell as claimed in claim 17 or 18,
**wherein**
oxidoreductases and, if desired, electron carriers are immobilized on the surface of a carrier material.

20. Cell as claimed in claim 19,
**wherein**
the solid carrier material consists of plastics, polysaccharides, silicates, silicon, graphite as well as derivatives and mixtures thereof.

21. Cell as claimed in claim 19 or 20,
**wherein**
the solid carrier material is in the form of fine particles.

22. Cell as claimed in claim 21,
**wherein**
the electrode body is present embedded in the carrier material.

23. Cell as claimed in claim 21,
**wherein**
the carrier material is present in the pores or holes of the electrode body.

24. Cell as claimed in claim 17 or 18,
**wherein**
oxidoreductases as well as, if desired, electron carriers are immobilized on the surface of the electrode body.

25. Cell as claimed in one of the claims 17 to 24,
**wherein**
it is a flow cell.

26. Cell as claimed in one of the claims 17 to 25,
**wherein**
it contains non-toxic dyes, in particular food dyes, as the electron carriers.

27. Use of a potentiometric cell as claimed in one of the claims 17 to 25 for converting reducible or oxidizable substances in drinking water or waste water.

28. Use as claimed in claim 27 for the conversion of nitrate.

29. Use as claimed in claim 27 for the conversion of methanol.

**30.** Use as claimed in claim 27 for the conversion of halogenated hydrocarbons.

**Revendications**

**1.** Procédé de transformation de substances réductibles ou oxydables en solution aqueuse, caractérisé en ce que l'on met en contact la solution aqueuse à traiter avec des oxydoréductases immobilisées, éventuellement en présence d'agents de transfert d'électrons co-immobilisés, et on fournit en même temps des équivalents de réduction ou des équivalents d'oxydation que l'on produit par électrolyse de l'eau.

**2.** Procédé selon la revendication 1, caractérisé en ce que l'on utilise des oxydoréductases et des agents de transfert d'électrons immobilisés sur un support solide dans un voisinage moléculaire immédiat et/ou directement liés les uns aux autres.

**3.** Procédé selon la revendication 2, caractérisé en ce que l'on choisit comme agent de transfert d'électrons un colorant non toxique.

**4.** Procédé selon l'une des revendications 1 à 3, caractérisé en ce que l'on fournit des équivalents de réduction ou d'oxydation susceptibles d'être régénérés, obtenus par électrolyse.

**5.** Procédé selon l'une des revendications 1 à 4, caractérisé en ce que l'on utilise comme équivalent de réduction de l'hydrogène atomique.

**6.** Procédé selon l'une des revendications 1 à 5, caractérisé en ce que l'on utilise comme équivalent d'oxydation de l'oxygène atomique.

**7.** Procédé selon la revendication 4, caractérisé en ce que l'on produit les équivalents de réduction ou d'oxydation dans une cellule potentiométrique qui contient les oxydoréductases immobilisées et éventuellement les agents de transfert d'électrons co-immobilisés, à une tension convenable, et on fait s'écouler en continu la solution à purifier à travers la cellule.

**8.** Procédé selon la revendication 7, caractérisé en ce que l'on règle le temps de séjour de la solution dans la cellule entre 0,2 et 5 volumes de cellule/min.

**9.** Procédé selon la revendication 7 ou 8, caractérisé en ce que l'on utilise une cellule à zones cathodique et anodique séparées et on réunit la solution qui s'écoule des deux zones.

**10.** Procédé selon l'une des revendications 7 à 9, caractérisé en ce que l'on utilise plusieurs cellules montées en série.

**11.** Procédé selon la revendication 10, caractérisé en ce que les cellules individuelles contiennent des oxydoréductases immobilisées différentes.

**12.** Procédé selon l'une des revendications précédentes, caractérisé en ce que l'on transforme en solution aqueuse des substances qui sont choisies dans le groupe constitué par
(a) des sels inorganiques, en particulier des nitrates, des sulfates, des cyanures, des sulfures et des phosphates, et
(b) des composés organiques, en particulier des hydrocarbures aliphatiques ou aromatiques, des hydrocarbures halogénés, des composés phospho-organiques, des alcools, des cétones, des phénols et des détergents.

**13.** Procédé selon la revendication 12, caractérisé en ce que l'on transforme des nitrates en solution aqueuse.

**14.** Procédé selon la revendication 12, caractérisé en ce que l'on transforme du méthanol en solution aqueuse.

**15.** Procédé selon la revendication 12, caractérisé en ce que l'on transforme des hydrocarbures halogénés en solution aqueuse.

**16.** Procédé selon l'une des revendications précédentes, caractérisé en ce que la concentration des substances à transformer en solution aqueuse est comprise entre environ 0,01 mmole/l et environ 10 mmoles/l.

17. Cellule potentiométrique pour la transformation de substances réductibles ou oxydables en solution aqueuse, caractérisée en ce qu'elle contient un corps d'électrode et des oxydoréductases immobilisées et éventuellement des agents de transfert d'électrons co-immobilisés.

18. Cellule selon la revendication 17, caractérisée en ce que les oxydoréductases et éventuellement les agents de transfert d'électrons sont immobilisés directement par formation de liaisons transversales ou par l'intermédiaire d'un espaceur à l'aide de composés époxy, bromure de cyanogène, diisocyanate, carbodiimide, glutaraldéhyde, EDC, FMP ou autres.

19. Cellule selon la revendication 17 ou 18, caractérisée en ce que les oxydoréductases et éventuellement les agents de transfert d'électrons sont immobilisés sur la surface d'un support.

20. Cellule selon la revendication 19, caractérisée en ce que le support solide est formé par des matières plastiques, des polysaccharides, des silicates, du silicium, du graphite et leurs dérivés et leurs mélanges.

21. Cellule selon la revendication 19 ou 20, caractérisée en ce que le support solide se présente sous une forme finement divisée.

22. Cellule selon la revendication 21, caractérisée en ce que en ce que le corps de l'électrode est enrobé dans le support.

23. Cellule selon la revendication 21, caractérisée en ce que le support se trouve dans des pores ou des orifices du corps de l'électrode.

24. Cellule selon la revendication 17 ou 18, caractérisée en ce que les oxydoréductases et éventuellement les agents de transfert d'électrons sont immobilisés sur la surface du corps de l'électrode.

25. Cellule selon l'une des revendications 17 à 24, caractérisée en ce qu'il s'agit d'une cellule à circulation.

26. Cellule selon l'une des revendications 17 à 25, caractérisée en ce qu'elle contient des colorants non toxiques, en particulier des colorants alimentaires, en tant qu'agents de transfert d'électrons.

27. Utilisation d'une cellule potentiométrique selon l'une des revendications 17 à 26 pour la transformation de substances réductibles ou oxydables dans l'eau potable ou les eaux usées.

28. Utilisation selon la revendication 27 pour la transformation de nitrates.

29. Utilisation selon la revendication 27 pour la transformation de méthanol.

30. Utilisation selon la revendication 27 pour la transformation d'hydrocarbures halogénés.

# Fig.1

$e^- +$  $H_3O +$  Elektronen-übertrüger  Enzym  Problemsubstanz

$(NO_3^-)$

$H$

$H_2O$  $H$  Enzym  reduzierter Zustand

Elektronen-übertrüger  $(NO_2^-)$

# Fig.2

$NO_2^-$  $NO_3^-$

$H$

$H$

$H$

$H$

Enzym  Elektronen-übertrüger

Elektrode

Trägermaterial

## Fig.3a

⊞ = Elektrodenkörper

⊡ = Enzym-haltige Matrix

## Fig.4a

## Fig.3b

## Fig.4b

# Fig.5a

🔲 = Elektrodenkörper
🔲 = Enzym-haltige Matrix

# Fig.6a

# Fig.5b

# Fig.6b

## Fig.7a

☑ = Elektrodenkörper
▦ = Enzym-haltige Matrix

## Fig.8a

## Fig.7b

## Fig.8b